# EUROPEAN PATENT APPLICATION

(11) **EP 4 279 179 A1**
(43) Date of publication of application: **22.11.2023**
(21) Application number: 22821839.2
(22) Date of filing: 15.06.2022
(51) Int. Cl.: B01L 3/00, B01L 7/00, G01N 21/64

(54) **FLUORESCENCE DETECTION CHIP, FLUORESCENCE DETECTION SYSTEM, FLUORESCENCE DETECTION METHOD, AND APPLICATION OF FLUORESCENCE DETECTION CHIP**

(30) Priority: 01.04.2022 CN 202210339877
(71) Applicant: Hefei Nuomaiji Biotech Co., Ltd, Hefei, China (Anhui) pilot Free Trade Zone Hefei Sity, Anhui 230093 (CN)
(72) Inventor: LIU, Yuchao, Anhui 241010 (CN)
(74) Representative: Dantz, Jan Henning
(86) International application number: PCT/CN2022/098877
(87) International publication number: WO 2023/184716

(57) **Abstract**

The present invention provides a fluorescence detection chip, a fluorescence detection system, a fluorescence detection method and an application thereof. The fluorescence detection chip comprises an upper casing and a lower casing, and further comprises a sample cell, a mixed liquid cell, a waste liquid cell and a number of independent reaction detection cells, wherein the reaction detection polls have cell walls made of a superconducting thermal material; wherein the sample cell, the mixed liquid cell, the waste liquid cell and the reaction detection cells are all arranged in the lower casing, and in the upper casing a number of feeding ports are provided, which correspond to the sample cell, the mixed liquid cell and the reaction detection cells, respectively. By means of a micro-channel design, the fluorescence detection chip realizes such a reaction effect that a reaction liquid is fully mixed during the flow process, and is evenly distributed to the respective reaction detection cells. The reaction detection cells are closed chambers formed by a superconducting thermal material and upper and lower casings to provide a high-intensity heat source for a reaction system to be detected, thereby speeding up the reaction speed, shortening the detection time, realizing the accurate quantitative detection, and having the advantages of convenient portability and convenient use.

## Description

### Technical field

The present invention relates to the technical field of microfluidic detection, and in particular relates to a fluorescence detection chip, a fluorescence detection system comprising the fluorescence detection chip, a fluorescence detection method based on the fluorescence detection chip, and an application of the fluorescence detection chip.

### Technical background

At present, in the field of medical molecular diagnosis, polymerase chain reaction (PCR) is an emerging technology that has sprung up suddenly, and it has emerged as a major mode in the field of diagnosis. Nowadays, various large-scale multi-functional PCR detection equipment can be seen everywhere, and has made outstanding contributions to the global fight against the epidemic. However, as a powerful detection method, how to achieve convenient detection and enter thousands of households is a challenge for contemporary scientific researchers. As a technology for precise control and manipulation of micro-scale fluids, microfluidic chip technology came into being. Microfluidics is mainly characterized by the manipulation of fluids in the micro-nano scale space, and has the ability to reduce the basic functions such as sample preparation, reaction, separation and detection of biological and chemical laboratories to a chip of a few square centimeters. Its basic feature and greatest advantage lie in the flexible combination and scale integration of various unit technologies on an overall controllable micro platform, involving the interdisciplinary research of multiple fields such as engineering, physics, chemistry, micro-processing and bioengineering.

The microfluidic detection chip is a detection platform based on microfluidic technology. It integrates basic operations such as sample preparation, reaction, and kit detection into a micron-scale chip to automatically complete the analysis process. Microfluidic detection chips generally have the advantages of less sample consumption, fast detection speed, easy operation, multi-functional integration, small size and portability.

However, the current microfluidic chip technology is still in its initial stage, and there are still many shortcomings: (1) it can only sort some sample fluids, extract some required molecular components, and then conduct other experimental verifications; and (2) predetermined detection reagents are simply mixed in the chip, and react in a conventional environment to observe their color change, which realizes some simple and conventional detection and judgments. It is difficult to realize accurate quantitative detection of existing large-scale equipment.

### Summary of the invention

In view of this, the present invention aims to provide a fluorescence detection chip. By means of a micro-channel design, this fluorescence detection chip realizes such a reaction effect that a reaction liquid is fully mixed during the flow process, and is evenly distributed to the respective reaction detection cells according to desired amounts. At the same time, the reaction detection cells adopt closed chambers formed by a superconducting thermal material and upper and lower casings to provide a high-intensity heat source for a reaction system to be detected, thereby speeding up the reaction speed, shortening the detection time, realizing the accurate quantitative detection of matching large-scale equipment, and having the advantages of convenient portability and convenient use.

In order to achieve the above objective, the present invention adopts the following technical solutions:
The present invention provides a fluorescence detection chip comprising an upper casing and a lower casing, and further comprising:
a sample cell for collecting a sample to be detected and lysing the sample to be detected;
a mixed liquid cell connected to the sample cell through a first micro-channel and used for extracting target fragments from the lysed sample to be detected;
a waste liquid cell connected to the mixed liquid cell, and used for collecting a waste liquid generated during the extraction process of the target fragments; and
a number of independent reaction detection cells connected to the mixed liquid cell through a second micro-channel and used for fluorescent detection of the target fragments, wherein the reaction detection cells have cell walls made of a superconducting thermal material;
wherein the sample cell, the mixed liquid cell, the waste liquid cell and the reaction detection cells are all arranged in the lower casing, and in the upper casing a number of feeding ports are provided, which correspond to the sample cell, the mixed liquid cell and the reaction detection cells, respectively.

In a further embodiment, the superconducting thermal material is selected from aluminum alloy, copper alloy or silver alloy.

In a further embodiment, in the sample cell a lysis absorbing and releasing member is provided, which is used to absorb and release the sample to be detected and a lysis buffer, so that they are fully contacted and lysed.

In a further embodiment, a feeding port of the sample cell is sealed with an anti-fouling member.

In a further embodiment, a number of first soft inserts and second soft inserts are provided on the surface of the upper housing;
wherein the first soft inserts are used to cut off the connection of the reaction detection cell with the mixed liquid cell and an external environment; and
the second soft inserts are arranged between adjacent reaction detection cells, and are used to block optical signal crosstalk between the reaction detection cells.

In a further embodiment, materials of the first soft insert and the second soft insert are independently selected from TPE, TPR, PU or silica gel and combinations thereof, and the second soft insert is made of a black or light-absorbing material.

In a further embodiment, the surface of the second soft insert is provided with an exhaust hole, and the exhaust hole is connected to a corresponding reaction detection cell.

The present invention further provides a fluorescence detection system comprising fluorescence detection equipment and the said fluorescence detection chip.

The present invention further provides a fluorescence detection method using the said fluorescence detection chip, characterized in that the fluorescence detection method comprises the following steps:
adding a sample to be detected and a lysis buffer to the sample cell to lyse the sample to be detected;
guiding a lysed sample mixture to be detected into the mixed liquid cell through the first micro-channel, and extracting target fragments from the sample to be detected by using a magnetic bead method;
evenly distributing and guiding the extracted target fragments to a corresponding reaction detection cell through the second micro-channel, and adding a diagnostic reagent to the reaction detection cell at the same time;
and carrying out heating, amplification and excited fluorescence reaction on the sample to be detected in the reaction detection cell for detecting a fluorescence signal.

The present invention further provides an application of the said fluorescence detection chip in medical diagnosis, scientific research experiments, import and export inspection and quarantine, forensic detection, animal and plant quarantine detection or disease control detection.

Compared with the prior art, the present invention has the following beneficial effects:
The fluorescence detection chip in the present invention is equipped with various reaction chambers of large-scale equipment on the chip structure, and integrates the processes of sampling, reaction, detection and so on. By means of the micro-channel design, the effect of fully mixing the reaction liquid in the flow process is realized, and the distribution of each reaction liquid to each reaction detection cell according to the desired amount is realized, thereby realizing the simultaneous detection of multiple items. The reaction detection cell adopts the superconducting thermal material and the upper and lower casing to form an independent detection space, which can provide a high-intensity heat source for the reaction system to be detected, thereby speeding up the reaction speed, and realizing rapid detection.

The fluorescence detection chip also has the advantages of compact appearance, convenient portability and convenient use.

### Brief description of the drawings

FIG. 1 is a schematic view of a three-dimensional structure of a fluorescence detection chip in Embodiment 1 of the present invention;
FIG. 2 is a schematic exploded view of the structure of the fluorescence detection chip in FIG. 1;
FIG. 3 is a schematic structural view of a lower casing 2 in FIG. 1;
FIG. 4 is a schematic structural view of an upper casing 1 in FIG. 1;
FIG. 5 is a schematic view of a partially enlarged structure of a reaction detection cell 24 in FIG. 1; and
FIG. 6 is a schematic exploded view of a structure of a fluorescence detection chip in Embodiment 2 of the present invention.

### List of reference signs

1 upper casing, 101 sample inlet, 102 detergent inlet, 103 eluent inlet, 104 pressure exhaust port, 105 magnetic bead liquid inlet, 106 first exhaust port, 107 diagnostic reagent inlet, 108 second exhaust port
2 lower casing, 21 sample cell, 211 lysis absorbing and releasing cotton, 212 anti-fouling sticker, 22 mixed liquid cell, 23 waste liquid cell, 24 reaction detection cell, 241 superconducting thermal chamber
3 middle casing;
401 first micro-channel, 402 second micro-channel
501 first soft insert, 502 second soft insert

### Detailed description of the embodiments

A fluorescence detection chip in the present invention will be further described in detail below with reference to the accompanying drawings.

It should be noted that when an element is referred to as being "fixed on", "disposed on", or "installed on" another element, it may be directly on said another element or indirectly on said another element. However, an element is referred to as being "connected to" or "connected with" another element, and it may be directly connected to said another element or indirectly connected to said another element. In addition, connection generally refers to fixing, and fixing here may be any one of conventional fixing methods in the field, such as "threaded connection", "riveting", "welding" and so on.

It should be understood that the orientation or positional relationships indicated by terms "length", "width", "upper", "lower", "front", "rear", "left", "right", "vertical", "horizontal", "top", "bottom", "inner", "outer", etc. are based on the orientation or positional relationships shown in the figures of the description, which is merely for the convenience of describing the embodiments of the present invention and simplifying the description, and does not indicate or imply that the mentioned device or element must have a particular orientation and be constructed or operated in the particular orientation. Therefore, the above-mentioned terms cannot be construed as a limitation of the present invention.

### Embodiment 1

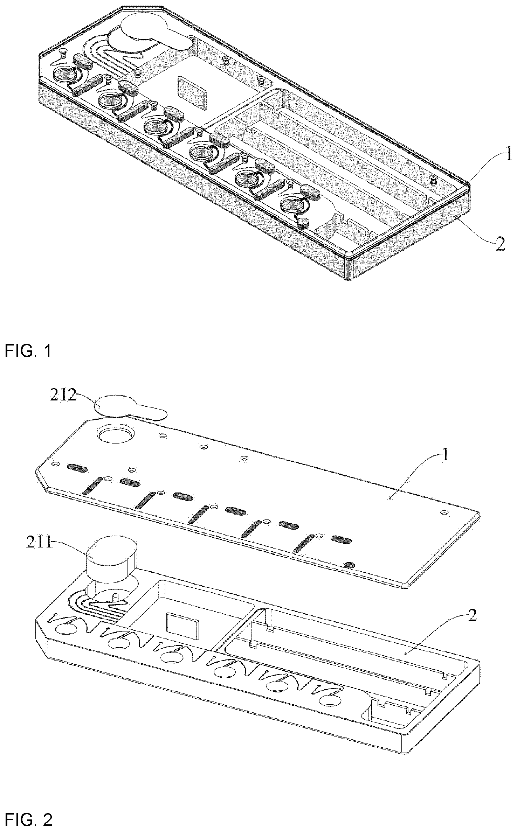

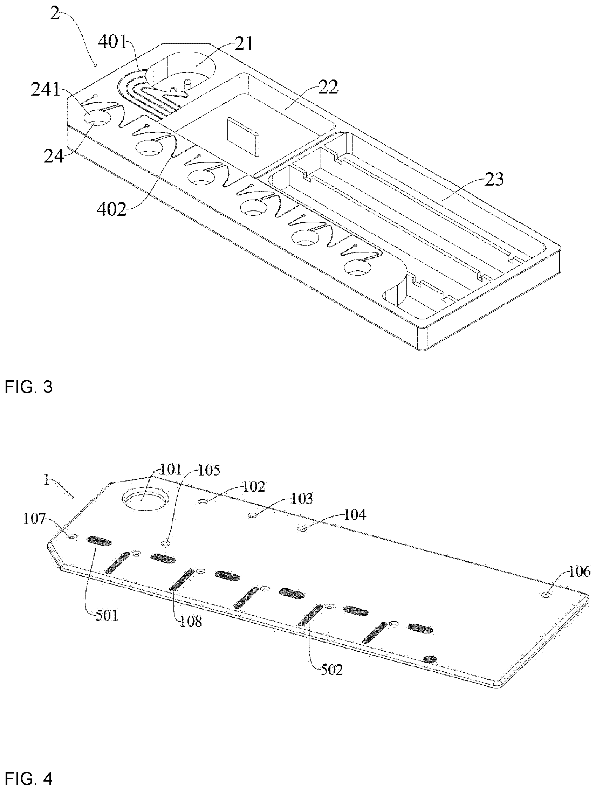

Please refer to FIGS. 1 and 2, which show a fluorescence detection chip. The fluorescence detection chip comprises an upper casing 1 and a lower casing 2, and the upper casing 1 and the lower casing 2 are bonded in a conventional bonding manner in the art to form the whole chip. The specific bonding process includes but is not limited to electrostatic bonding, thermocompression bonding or laser bonding, and it is not particularly limited. The materials of the upper casing 1 and the lower casing 2 can be selected conventionally in the art, such as high light-transmitting plastics, metal alloys, non-metallic or other mixed materials. It should be particularly noted that, in order to achieve the purpose of fluorescence detection, the upper casing 1 must be made of a high light-transmitting material, whereas the lower casing 2 is not particularly limited. Any material may be used for the lower casing 2 as long as it meets the requirement of good biocompatibility. Further, with reference to FIG. 3, in this embodiment, a sample cell 21, a mixed liquid cell 22, a waste liquid cell 23 and a number of reaction detection cells 24 are provided in the lower casing 2, wherein the number of reaction detection cells 24 is not particularly limited, and there is at least one. The specific number can be adjusted according to actual needs and the chip size, such as 2, 3, 4, 6, 8. In the chip structure of this embodiment, six reaction detection cells 24 are provided in the lower casing 2. In addition, with reference to FIGS. 2 and 4, a plurality of feeding ports are provided in the upper casing 1, and correspond to the sample cell 21, the mixed liquid cell 22, the waste liquid cell 23 and the reaction detection cells 24, respectively. The specific number and positions of feeding ports can be adjusted according to the positions of the respective reaction cells and needs.

Please continue to refer to FIG. 3. Specifically, the sample cell 21 is used to collect a sample to be detected and to lyse the sample to be detected. As shown in FIGS. 1 and 2, a lysis absorbing and releasing cotton 211 is provided in the sample cell 21. This lysis absorbing and releasing cotton 211 has a hydrophilic effect, and is used for absorbing the sample to be detected and a lysis buffer, so that the sample to be detected is fully contacted with the lysis buffer and lysed. Preferably, the lysis absorbing and releasing cotton 211 may also have a lysing effect, so that the sample to be detected is lysed more fully. The fixing manner of the lysis absorbing and releasing cotton 211 is not particularly limited. Specifically, as shown in FIG. 2, in this embodiment, a column is provided in the sample cell 21, and the lysis absorbing and releasing cotton 211 is inserted on the column to realize fixing. Further, with reference to FIGS. 2 and 4, a sample inlet 101 is provided in the upper casing 1 corresponding to the position of the sample cell 21, and the sample to be detected and the lysis buffer can be added into the sample cell 21 through the sample inlet 101 to obtain a lysed sample mixture.

In this embodiment, a further preferred modification may be made. That is, in addition to the sample inlet 101 in the upper casing 1 corresponding to the position of the sample cell 21, a lysis buffer inlet is also provided, so that the sample to be detected and the lysis buffer are added into the sample cell 21 through different feeding ports, respectively, which can prevent the inlet of the supporting equipment for adding the lysis buffer from being contaminated by the sample to be detected.

Further, as shown in FIGS. 1 and 2, the feeding port (the sample inlet 101 and/or the lysis buffer inlet) of the sample cell 21 is sealed with an anti-fouling sticker 212, and the anti-fouling sticker 212 seals the feeding port of the sample cell 21. On the one hand, the lysis absorbing and releasing cotton 211 fixed in the sample cell 21 is prevented from being contaminated by the outside. On the other hand, after the sampling is completed, the feeding port of the sample cell 21 can be sealed once again to avoid interference from the external environment, thereby ensuring the accuracy of the reaction. It should be noted that the sticky side of the anti-fouling sticker 212 cannot react with the lysis buffer and the sample to be detected, etc.

Further, please continue to refer to FIG. 3. In this embodiment, the mixed liquid cell 22 is connected to the sample cell 21 through a first micro-channel 401. The mixed liquid cell 22 is used for further lysis of the sample mixture to be detected and extracting target fragments from the lysed sample mixture to be detected. The number of first micro-channels 401 is not particularly limited, and there is at least one. Preferably, there may be multiple channels, such as 4, 5. The sample cell 21 and the mixed liquid cell 22 are connected by designing multiple first micro-channels 401, so that the sample mixture to be detected in the sample cell 21 is continuously lysed during the process of entering the mixed liquid cell 22 through the first micro-channels 401, thereby ensuring sufficient lysing. In this embodiment, the magnetic bead method is used in the mixed liquid cell 22 to extract the target fragments from the sample to be detected. With reference to FIG. 4, a detergent inlet 102, an eluent inlet 103, a pressure exhaust port 104, and a magnetic bead liquid inlet 105 are provided in the upper housing 1 corresponding to the position of the mixed liquid cell 22, respectively. A detergent, an eluent and a magnetic bead liquid as required are added into the mixed liquid cell 22 through the detergent inlet 102, the eluent inlet 103 and the magnetic bead liquid inlet 105, and the pressure exhaust port 104 is used to pressurize the mixed liquid cell 22 so as to drive the flow of the fluid through pressure.

Further, please continue to refer to FIG. 3, and the waste liquid cell 23 communicates with the mixed liquid cell 22 through micro-channels. The number of communicated micro-channels is not particularly limited. The waste liquid cell 23 is used to collect waste liquid generated during the extraction process of the target fragments in the mixed liquid cell 22. Specifically, with reference FIG. 4, in the upper casing 1 corresponding to the position of the waste liquid cell 23 a first exhaust port 106 is provided, which is used to discharge the gas generated during the reaction process in the chip and discharged during the process of guiding the waste liquid to the waste liquid pool.

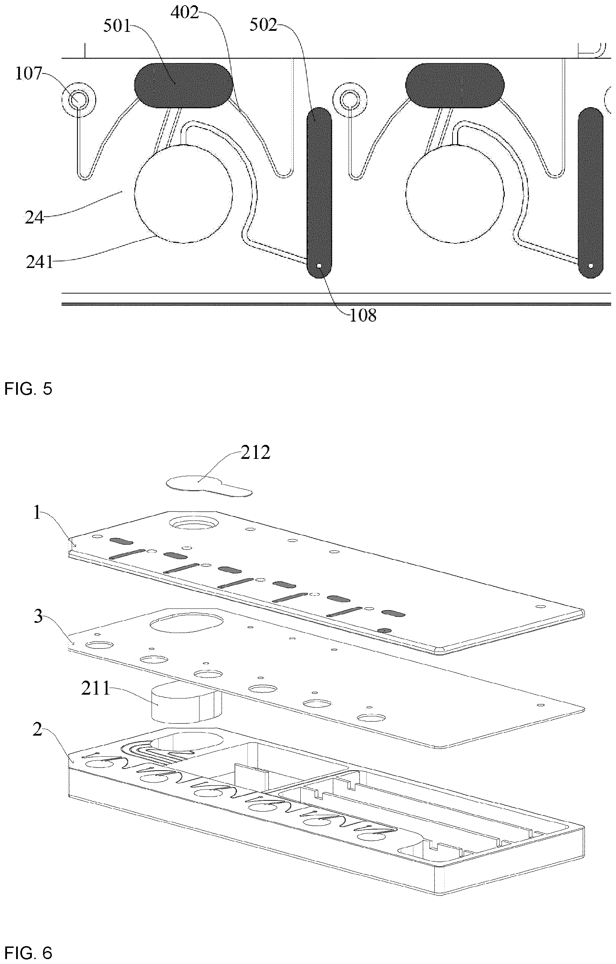

Further, as shown in FIG. 3, in this embodiment, there are six reaction detection cells 24. The structures of the reaction detection cells 24 are all the same. Specifically, the reaction detection cells 24 are connected to the mixed liquid cell 22 through corresponding second micro-channels 402, respectively, so that the extracted target fragments are evenly distributed through the second micro-channels 402 and then enter the corresponding reaction detection cells 24. In order to ensure that the target fragments are evenly distributed into the respective reaction detection cells 24, the degree of curvature of the second micro-channels 402 is as consistent as possible, and the flow resistance of each channel is the same. Further, with reference to FIG. 4, the upper casing 1 is provided with diagnostic reagent inlets 107 corresponding to the reaction detection cells 24, and the diagnostic reagent inlets 107 are connected to the corresponding reaction detection cells 24 through micro-channels. A diagnostic reagent is added to corresponding reaction detection cells 24 through the diagnostic reagent inlets 107 to react with the target fragments for fluorescence detection. Further, as shown in FIG. 3, the cell wall of the reaction detection cell 24 in this embodiment is a superconducting thermal chamber 241. Specifically, a groove is provided on the surface of the lower casing 2, and the superconducting thermal chamber 241 is embedded in the groove. The upper casing 1 and the lower casing 2 are bonded so that the superconducting thermal chamber 241 forms independent reaction chambers with the upper casing 1 and the lower casing 2 separately. The superconducting thermal chamber 241 is made of a superconducting thermal material, and the superconducting thermal material is a metal or alloy that has a thermal conductivity > 200 W/m•°C and is inert to reactants in the reaction detection cells 24. The inert means that the material does not react with the reactants in the reaction detection cells 24. Specifically, it can be realized by carrying out an inert oxidation treatment on the surface of the material. The superconducting thermal material may be selected from aluminum alloy, silver alloy or copper alloy. It should be noted that the superconducting thermal chamber 241 also needs to be treated with black light absorption to ensure the required reaction environment for fluorescence detection. Further, please refer to FIG. 4, and a number of first soft inserts 501 and second soft inserts 502 are separately provided in the upper casing 1. Specifically, the upper casing 1 is provided with holes for accommodating the first soft inserts 501 and the second soft inserts 502, so that the first soft inserts 501 and the second soft inserts 502 are embedded in the upper casing 1. With reference to FIGS. 1 and 5, the first soft inserts 501 correspond to the positions of the second micro-channels 402 of the reaction detection cells 24 and the micro-channels of the diagnostic reagent inlets 107; and the second soft inserts 502 are provided between adjacent reaction detection cells 24. A second exhaust port 108 is provided on the surface of the second soft insert 502, and the second exhaust port 108 communicates with the corresponding reaction detection cell 24 through a micro-channel. Through the cooperation of external supporting equipment, pressure is applied to the first soft insert 501, so that the first soft insert 501 is pressed down, and thus the second micro-channel 402 and the micro-channel of the diagnostic reagent inlet 107 are cut off. Also, the external supporting equipment blocks the second exhaust port 108 in the second soft insert 502, so that the corresponding reaction detection cell 24 becomes a closed independent space, thereby cutting off the passage between the reaction detection cell 24 and the outside and the mixed liquid cell 22, which can avoid aerosol pollution generated during the reaction detection process. In this embodiment, the first soft insert 501 and the second soft insert 502 are made of soft plastic or colloid, and specific examples include but are not limited to TPE, TPR, PU or silica gel and combinations thereof. The use of these soft materials can well realize the airtightness of the reaction detection cells 24. In addition, in this embodiment, the second soft insert 502 is also used for isolating optical signals to avoid cross-light interference when adjacent reaction detection cells 24 perform fluorescence detection. Therefore, the second soft insert 502 is made of a black or light-absorbing material.

The fluorescence detection chip described in this embodiment can detect multiple items simultaneously for one sample, and its specific working flow is as follows:
A feedstock liquid of a sample to be detected is dropped from the sample inlet 101 onto the lysis absorbing and releasing cotton 211 of the sample cell 21. Then, the fluorescence detection chip is inserted into a supporting equipment, and a running program is set to start running. The supporting equipment first presses a sample lysis buffer into the lysis absorbing and releasing cotton 211 through the sample inlet 101 or the lysis buffer inlet, and the sample to be detected is lysed. The lysed sample mixture flows into the mixed liquid cell 22 through the first micro-channel 401, and during the process of entering the mixed liquid cell 22, the sample to be detected is continuously lysed.

When the sample mixed liquid has just flowed into the mixed liquid cell 22, the magnetic bead liquid inlet 105 can be opened to add a magnetic bead liquid into the mixed liquid cell 22. After specified amounts of the two liquids are added, the supporting equipment provides a vibration function and a heating function to facilitate the full reaction between the lysis buffer and the sample to be detected, so that the DNA fragments are fully released and combined with the magnetic beads. After this procedure is completed, an electromagnetic strip of auxiliary supporting equipment is energized, and the magnetic beads in the mixed liquid cell 22 are adsorbed on the periphery of the electromagnetic strip. At this time, the pressure exhaust port 104 arranged above the mixed liquid cell 22 is pressurized (at this time, the second exhaust port 108 is in a blocked state, and the first exhaust port 106 is in an open state). Under the driving of pressure, waste liquid is discharged into the waste liquid cell 23 through the micro-channels. After the waste liquid is completely discharged, a predetermined amount of eluent is added to the mixed liquid cell 22 from the eluent inlet 103. At this time, the electromagnetic strip is turned off, and the supporting equipment provides vibration to facilitate the separation of the DNA fragments from the magnetic beads. Then, the electromagnetic strip is turned on once again to adsorb the magnetic beads. The pressure exhaust port 104 arranged above the mixed liquid cell 22 works once again to make DNA sample liquid in the mixed liquid cell 22 evenly distributed and added to the respective reaction detection cells 24 provided in the lower casing 2 (specifically, the first exhaust port 106 is in a closed state, the sample inlet 101 and the lysis buffer inlet (if any) are also in a closed state, one of the second exhaust ports 108 is opened, the remaining second exhaust ports 108 are blocked, and then a pressure is applied so that the DNA sample liquid in the mixed liquid cell 22 enters the corresponding reaction detection cell 24 with the second exhaust port 108 opened, and the remaining reaction detection cells 24 are operated in the same way). At the same time, the supporting equipment will also add predetermined amounts of diagnostic reagents into corresponding reaction detection cells 24 through the diagnostic reagent inlets 107 according to the requirements of the detection items.

After the above process is finished, the supporting equipment presses the first soft insert 501 to cut off the second micro-channels 402 and the channels of the diagnostic reagent inlets 107 and block all the second exhaust outlets 108, so that the respective reaction detection cells 24 form a completely closed independent reaction chamber. At the same time, the supporting equipment heats the reaction detection cells 24 according to the set program. In addition, supporting equipment is equipped with surface probes corresponding to the reaction detection cells 24, reaction signals of the sample to be detected in the reaction detection cells 24 in the process of each temperature cycle are clearly and actually recorded and stored, and then detection results are output through an algorithm software system to complete the detection. It can be understood that the supporting equipment described in this process is an automatic instrument, and it can be programmed to cooperate with the fluorescence detection chip to complete the entire detection process, which will not be described in detail here.

This embodiment further provides a fluorescence detection system, which comprises at least fluorescence detection equipment and the above-mentioned fluorescence detection chip, and may further comprise some automated operating equipment, control and result analysis modules, etc. The fluorescence detection equipment can be used in conjunction with the fluorescence detection chip. Specific examples include but are not limited to fluorescence indicators, fluorescence detectors, etc.; and the result analysis module includes but is not limited to computers and supporting operation and analysis software, etc.

Based on the fluorescence detection chip provided in this embodiment, the present invention further discloses a fluorescence detection method, comprising the following steps:
adding a sample to be detected and a lysis buffer to the sample cell to lyse the sample to be detected;
guiding a lysed sample mixture to be detected into the mixed liquid cell through the first micro-channel, and extracting target fragments from the sample to be detected by using a magnetic bead method;
evenly distributing and guiding the extracted target fragments to a corresponding reaction detection cell through a second micro-channel, and adding a diagnostic reagent to the reaction detection cell at the same time;
and carrying out heating, amplification and excited fluorescence reaction on the sample to be detected in the reaction detection cell for detecting a fluorescence signal.

### Embodiment 2

In addition to the chip structure formed by the upper casing 1 and the lower casing 2, the fluorescence detection chip in the present invention may also be provided with a multi-layer stacked structure (such as 3 layers, 4 layers) of at least one layer of casings in the upper casing 1 and the lower casing 2. By setting a multi-layer stacked structure, the micro-channels or reaction chambers can be arranged in layers, so as to reasonably optimize the internal structure of the chip, so that the micro-channels do not interfere with each other, and can communicate with each other when necessary. The specific structure can be adjusted according to actual situations.

FIG. 6 shows an optimized chip structure on the basis of Embodiment 1. It further comprises a middle casing 3. A number of through holes are provided in the middle casing 3, and the micro-channels of the respective reaction chambers are reasonably distributed through these through holes to avoid mutual interference of the micro-channels.

### Embodiment 3

The fluorescence detection chip in the present invention may also detect multiple samples at the same time. Specifically, the structure of Embodiment 2 is transformed so that it comprises an upper casing 1, a lower casing 2 and a middle casing 3. Two sample cells 21 and two mixed liquid cells 22 (the sample cells 21 and the mixed liquid cells 22 are in a one-to-one correspondence relationship) are provided in the lower casing 2, wherein one of the mixed liquid cells 22 is connected to three reaction detection cells 24 through second micro-channels 402, respectively; and the other of the mixed liquid cells 22 is connected to another three reaction detection cells 24 through second micro-channels 402, respectively. Through the design of through holes in the middle casing 3, the second micro-channels 402 are separated to avoid mutual interference. Other structures and processes are similar with Embodiments 1 and 2, and will not be described in detail here.

The fluorescence detection chip in this embodiment can perform multi-item detection on two samples at the same time, which is convenient and fast. It can be understood that there may be a plurality of sample cells 21 and mixed liquid cells 22 (such as 3, 4), which can be adjusted according to actual needs and the chip size, and will not be described in detail here.

The technical features of the above embodiments can be combined arbitrarily. In order to make the description concise, all possible combinations of various technical features in the above embodiments are not described. However, as long as there is no contradiction in the combination of these technical features, all of these should be considered to be within the scope set forth in the description.

The above embodiments only represent several implementations of the present invention, and the description thereof is relatively specific and detailed, but it should not be interpreted as limiting the scope of the patent of invention. It should be noted that, for an ordinary person skilled in the art, several modifications and improvements can be made without departing from the concept of the present invention, and all of these fall within the scope of protection of the present invention. Therefore, the scope of protection of the present patent for invention should be defined by the appended claims.

## Claims

1. A fluorescence detection chip, comprising an upper casing and a lower casing, **characterized in that** the fluorescence detection chip further comprises:
a sample cell for collecting a sample to be detected and lysing the sample to be detected;
a mixed liquid cell connected to the sample cell through a first micro-channel and used for extracting target fragments from the lysed sample to be detected;
a waste liquid cell connected to the mixed liquid cell, and used for collecting a waste liquid generated during the extraction process of the target fragments;
and a number of independent reaction detection cells connected to the mixed liquid cell through a second micro-channel and used for fluorescent detection of the target fragments, wherein the reaction detection cells have cell walls made of a superconducting thermal material;
wherein the sample cell, the mixed liquid cell, the waste liquid cell and the reaction detection cells are all arranged in the lower casing, and in the upper casing a number of sample inlets are provided, which correspond to the sample cell, the mixed liquid cell and the reaction detection cells, respectively.

2. The fluorescence detection chip according to claim 1, wherein the superconducting thermal material is selected from aluminum alloy, copper alloy or silver alloy.

3. The fluorescence detection chip according to claim 1, wherein in the sample cell a lysis absorbing and releasing member is provided, which is used to absorb and release the sample to be detected and a lysis buffer, so that they are fully contacted and lysed.

4. The fluorescence detection chip according to claim 1, wherein a feeding port of the sample cell is sealed with an anti-fouling member.

5. The fluorescence detection chip according to claim 1, wherein a number of first soft inserts and second soft inserts are provided on the surface of the upper housing;
wherein the first soft inserts are used to cut off the connection of the reaction detection cell with the mixed liquid cell and an external environment; and the second soft inserts are arranged between adjacent reaction detection cells, and are used to block optical signal crosstalk between the reaction detection cells.

6. The fluorescence detection chip according to claim 5, wherein materials of the first soft insert and the second soft insert are independently selected from TPE, TPR, PU or silica gel and combination thereof, and the second soft insert is made of a black or light-absorbing material.

7. The fluorescence detection chip according to claim 5 or 6, wherein the surface of the second soft insert is provided with an exhaust port, which is connected to a corresponding reaction detection cell.

8. A fluorescence detection system, comprising a fluorescence detection device, **characterized in that** the fluorescence detection system further comprises the fluorescence detection chip of any one of claims 1-7.

9. A fluorescence detection method, which adopts the fluorescence detection chip of any one of claims 1-7, **characterized in that** it comprises the following steps:
adding a sample to be detected and a lysis buffer to the sample cell to lyse the sample to be detected;
guiding a lysed sample mixture to be detected into the mixed liquid cell through the first micro-channel, and extracting target fragments from the sample to be detected by using a magnetic bead method;
evenly distributing and guiding the extracted target fragments to a corresponding reaction detection cell through the second micro-channel, and adding a diagnostic reagent to the reaction detection cell at the same time;
and carrying out heating, amplification and excited fluorescence reaction on the sample to be detected in the reaction detection cell for detecting a fluorescence signal.

10. Application of the fluorescence detection chip of any one of claims 1-7 in medical diagnosis, scientific research experiments, import and export inspection and quarantine, forensic detection, animal and plant quarantine detection or disease control detection.
